# EUROPEAN PATENT APPLICATION

(11) **EP 4 342 445 A1**
(43) Date of publication of application: **27.03.2024**
(21) Application number: 22804256.0
(22) Date of filing: 07.02.2022
(51) Int. Cl.: A61K 8/73, A61K 8/04, A61K 8/29, A61K 8/27, A61K 8/99, A61Q 19/00, A61Q 17/04

(54) **AQUEOUS DISPERSION**

(30) Priority: 18.05.2021 JP 2021083629
(71) Applicant: DIC Corporation, Tokyo 174-8520 (JP)
(72) Inventor: NONOKAWA Daigo, Sakura-shi Chiba 285-8668 (JP); KANEKO Maiko, Kumamoto-shi, Kumamoto 861-8038 (JP)
(74) Representative: TBK
(86) International application number: PCT/JP2022/004711
(87) International publication number: WO 2022/244334

(57) **Abstract**

The technical problem to be solved by the invention is to provide an aqueous dispersion which has an excellent moisture retaining property and excellent dispersion stability and can be used for various applications such as a cosmetic. It was found that the aqueous dispersion including a polysaccharide (A) having a sulfate group and a hydrophilization treatment-inorganic powder (B) has an excellent moisture retaining property and excellent dispersion stability and can be used for various applications such as a cosmetic.

## Description

### TECHNICAL FIELD

The present invention relates to an aqueous dispersion having excellent dispersion stability and a cosmetic containing thereof.

### BACKGROUND ART

In the field of cosmetics and the like, various inorganic powders are used as colorants, fillers, ultraviolet absorbers, and ultraviolet scattering agents. Although these inorganic powders are used in various applications as described above, there is a problem in that precipitation occurs when they are added to a liquid. Therefore, when these inorganic powders are used for formulation, various studies have been conducted, such as selection of a solvent having good affinity, study of a composition for improving dispersibility, and surface treatment of an inorganic powder for improving affinity (see, for example, Patent Literatures 1 to 3).

However, even when a dispersion is produced by combining a polysaccharide, which is known to have a thickening property and contribute to dispersion stability, with a hydrophilization treatment-inorganic powder, pseudo-crosslinking occurs due to interaction between the polysaccharide and the hydrophilization treatment-inorganic powder, and gelation occurs to make the dispersion system unstable, resulting in precipitation of the inorganic powder and separation into two layers, and the production of an aqueous dispersion having excellent dispersibility by combining these polysaccharides with a hydrophilization treatment-inorganic powder has not been achieved to date.

### CITATION LIST

### PATENT LITERATURE

PTL 1: JP2020-2031A
PTL2: JP2018-172303A
PTL3: JP2014-214128A

### SUMMARY OF INVENTION

### TECHNICAL PROBLEM

The technical problem to be solved by the invention is to provide an aqueous dispersion which has an excellent moisture retaining property and excellent dispersion stability and can be used for various applications such as a cosmetic.

### SOLUTION TO PROBLEM

The inventors solved the problem by the aqueous dispersion including a polysaccharide (A) having a sulfate group and a hydrophilization treatment inorganic powder (B).

That is, the invention includes the following aspects.
[1] An aqueous dispersion including a polysaccharide (A) having a sulfate group and a hydrophilization treatment-inorganic powder (B).
[2] The aqueous dispersion according to [1], in which the polysaccharide (A) having a sulfate group is a polysaccharide having a weight-average molecular weight of 3,500,000 to 20,000,000.
[3] The aqueous dispersion according to [1] or [2], in which the hydrophilization treatment is a hydrophilization treatment of coating a part or all of a surface of the inorganic powder with one or more surface treatment agents selected from silica, hydrous silica, hydrous alumina, zirconia, and the like.
[4] The aqueous dispersion according to any of [1] to [3], in which the inorganic powder is one or more selected from titanium oxide or zinc oxide.
[5] The aqueous dispersion according to any of [1] to [4], in which the hydrophilization treatment-inorganic powder (B) is contained in a range of 10 to 200 parts by weight with respect to 1 part by weight of the polysaccharide (A) having a sulfate group in the aqueous dispersion.
[6] The aqueous dispersion according to any of [1] to [5], in which the polysaccharide (A) having a sulfate group is a polysaccharide obtained from Suizenji Nori.
[7] A cosmetic including the aqueous dispersion according to any of [1] to [6].
[8] The cosmetic according to [7], in which the cosmetic is a sunscreen cosmetic or a moisturizing cosmetic.

### ADVANTAGEOUS EFFECTS OF INVENTION

According to the invention, an aqueous dispersion having excellent dispersion stability and excellent coating film formability can be produced, and the aqueous dispersion can be widely used in applications such as a cosmetic.

### DESCRIPTION OF EMBODIMENTS

Hereinafter, the detail of the invention is described. It should be noted that the description of the constituent features described below is an example for describing the invention, and the invention is not limited to these contents.

The polysaccharide (A) having a sulfate group used in the invention is not particularly limited within a range in which the effect of the invention can be obtained as long as it has a sulfate group in a part of the polysaccharide. As the polysaccharide (A) having a sulfate group, a polysaccharide having a sulfate group extracted from various raw materials may be used as it is, or a polysaccharide obtained by introducing a sulfate group into a polysaccharide having no sulfate group by a known method may be used. In addition, as the polysaccharide (A) having a sulfate group in the structure used in the invention, salts of these polysaccharides may also be used.

As the polysaccharide (A) having a sulfate group used in the invention, a polysaccharide having a sulfate group derived from blue-green algae is preferable, and in particular, a polysaccharide (SACRAN) having a sulfate group obtained from Suizenji Nori (scientific name: Aphanothece sacrum) is preferably used because the dispersion stability is improved.

In the invention, Suizenji Nori, which is suitable as a raw material of the polysaccharide (A) having a sulfate group, is a blue-green alga and is one kind of eubacteria belonging to the order Chroococcales and having photosynthesizing ability. The Suizenji Nori is a freshwater blue-green alga which grows naturally in a specific region of Kyushu and in which a plurality of cell form a flat colony. In addition, the outer surface of the colony is covered with a gelatinous secretion formed by polysaccharides and the like, and the diameter of the colony grows to about the size of 50 mm.

The method for extracting the polysaccharides (A) having a sulfate group from Suizenji Nori is not particularly limited, and a known method can be used for extraction, and examples thereof include a method described in Examples of WO2008/062574.

Examples of a specific method for extracting the polysaccharide (A) having a sulfate group from Suizenji Nori include the followings. An appropriate amount of Suizenji Nori is frozen and washed with water to remove a water-soluble pigment, and then a lipid soluble pigment is removed with an organic solvent such as ethanol, and the alga body of Suizenji Nori is dried. Then, 0.1 N of sodium hydroxide is added to the obtained dried alga body of Suizenji Nori, and stirring is performed at 60 to 80 °C for 6 hours. The sugar derivative solution obtained by the above operation is neutralized and then filtered, and further treated with a dialysis membrane having a molecular weight cut off of 8,000, and the inner liquid of the dialysis membrane is concentrated and dried.

As the polysaccharide (A) having a sulfate group used in the invention, in addition to the polysaccharide derived from Suizenji Nori, one or more kinds selected from the group consisting of chondroitin sulfate, dermatan sulfate and salts thereof; and one or more kinds selected from the group consisting of polysaccharides having a sulfate group in a part of known polysaccharides composed of D-glucosamine, D-galactosamine, D-glucuronic acid, L-iduronic acid, D-galacturonic acid, D-glucose, D-galactose, D-xylose and the like, and salts thereof can be used, and these components may be used alone or in combination of two or more kinds thereof appropriately.

The weight-average molecular weight of the polysaccharide (A) having a sulfate group used in the invention is not particularly limited as long as the effects of the invention can be obtained, and is preferably 3,500,000 to 20,000,000 because the dispersibility of the aqueous dispersion is stabilized, and more preferably 4,000,000 to 15,000,000 because the viscosity of the aqueous dispersion becomes suitable.

In the aqueous dispersion according to the invention, the content of the polysaccharide (A) having a sulfate group is not particularly limited as long as the effect of the invention can be obtained, but is preferably 0.005 to 1 % by weight, and more preferably 0.01 to 0.8 % by weight, with respect to the total aqueous dispersion, because the coating film formability of the aqueous dispersion becomes suitable.

The hydrophilization treatment-inorganic powder (B) used in the invention may be an inorganic powder obtained by surface-treating a known inorganic powder with a hydrophilic substance, and is not particularly limited as long as the effects of the invention can be obtained.

As the inorganic powder constituting the hydrophilization treatment-inorganic powder (B) of the invention, various known inorganic powders can be used, and for example, one or more kinds selected from inorganic powders such as zinc, titanium, calcium, aluminum, silica, boron, barium, talc, mica, kaolin and alumina, and oxides, hydroxides, carbonates, sulfates and nitrides thereof may be used. As the inorganic powder, an oxide is preferably used because the dispersibility is improved, and in particular, one or more kinds of zinc oxide and titanium oxide are preferably used because the dispersibility is significantly improved.

The hydrophilization treatment performed to convert the inorganic powder into the hydrophilization treatment-inorganic powder (B) of the invention is not particularly limited as long as the effects of the invention can be obtained, and may be any treatment that improves the affinity between the inorganic powder and water. Specific examples of the hydrophilization treatment include a treatment in which a part or all of the surface of the inorganic powder is coated with one or more surface treatment agents selected from silica, hydrous silica, hydrous alumina, zirconia, and the like, and among these, the hydrophilization treatment using silica or hydrous silica is preferable because the dispersibility is further improved. In addition, the hydrophilization treatment may be performed so as to form a multilayer.

The hydrophilization treatment is preferably performed by adding 1 to 35 parts by weight of a surface treatment agent with respect to 100 parts by weight of the inorganic powder before the treatment, and 2 to 25 parts by weight is more preferable because the dispersibility becomes more suitable.

In the aqueous dispersion of the invention, the blending ratio of the polysaccharide (A) having a sulfate group and the hydrophilization treatment-inorganic powder (B) is not particularly limited as long as the effect of the invention can be obtained, but the hydrophilization treatment-inorganic powder (B) is preferably contained in a range of 10 to 200 parts by weight, particularly preferably 20 to 150 parts by weight, with respect to 1 part by weight of the polysaccharide (A) having a sulfate group, because significant improvement in dispersibility is observed.

The aqueous dispersion of the invention can be suitably used in various applications where dispersibility is required. Especially, because a moisture retaining property derived from the polysaccharide can be suitably obtained when used as a cosmetic, a moisturizing cosmetic is preferable. Further, since the hydrophilization treatment-inorganic powder (B) has a suitable ultraviolet protection effect, a sunscreen cosmetic is preferable.

When the aqueous dispersion of the invention is used as a cosmetic, any components that can be used in the field of cosmetics, such as any oil agents, moisturizing agents, surfactants, ultraviolet protectors, plant and animal derived extracts, higher alcohols, antioxidants, coloring materials, pH adjusters, perfumes, preservatives, polysaccharides other than the component (A), and powders other than the component (B), may be added. When the aqueous dispersion of the invention is used as a cosmetic, the dosage form thereof is not particularly limited, and various dosage forms such as skin toner, lotion, cream and the like can be used, and these can also be used as basic cosmetics, makeup cosmetics and the like.

The dispersion method for obtaining the aqueous dispersion of the invention is not particularly limited as long as it is a known method capable of dispersing uniformly. Examples of the dispersion method include a stirrer, a planetary centrifugal mixer, a homomixer, a homodisper, an ultrasonic cleaner, a paint mixer (a sand mill), a ball mill, a roll mill, and the like.

### EXAMPLES

Hereinafter, the invention is described in more detail with reference to Examples, but the invention is not limited to these Examples. The weight-average molecular weights of the polysaccharides used in Examples and Comparative Examples were estimated values measured by multi-angle scattering gel permeation chromatography.

### <Preparation of Aqueous Dispersion>

### (Example 1)

30 parts by weight of hydrous silica-treated titanium oxide fine particles (manufactured by Sakai chemical industry Co., Ltd., STR-100W), 0.25 parts by weight of SACRAN (weight-average molecular weight (Mw) of about 4,000,000), 69.75 parts by weight of water, and zirconia beads having 4 times the weight of titanium oxide as a stirrer were placed in a polypropylene container with a lid (100 mL), covered with the lid, and then dispersed for 2 hours with the paint conditioner (manufactured by Toyo Seiki Seisaku-sho, Ltd.). Thereafter, Aqueous Dispersion 1 was obtained by filtering through a 200-micron polyethylene mesh. After the obtained Aqueous Dispersion 1 was allowed to stand at room temperature, the dispersion state over time was visually observed. As a result, Aqueous Dispersion 1 was in a uniform state even after 3 months. Further, when this Aqueous Dispersion 1 was applied to a latex sheet, a uniform coating film was obtained.

### (Example 2)

30 parts by weight of hydrous silica-treated titanium oxide fine particles (manufactured by Sakai chemical industry Co., Ltd., STR-100W), 0.25 parts by weight of SACRAN (weight-average molecular weight (Mw) of about 12,000,000), 69.75 parts by weight of water, and zirconia beads having 4 times the weight of titanium oxide as a stirrer were placed in a polypropylene container with a lid (100 mL), covered with the lid, and then dispersed for 2 hours with the paint conditioner. Thereafter, Aqueous Dispersion 2 was obtained by filtering through a 200-micron polyethylene mesh. The obtained Aqueous Dispersion 2 was allowed to stand at room temperature, and then the dispersion state over time was visually observed. As a result, Aqueous Dispersion 2 was in a uniform state even after 3 months. Further, when this Aqueous Dispersion 2 was applied to a latex sheet, a uniform coating film was obtained.

### (Example 3)

30 parts by weight of hydrous silica-treated zinc oxide fine particles (manufactured by Sakai chemical industry Co., Ltd., Finex52), 0.35 parts by weight of SACRAN (weight-average molecular weight (Mw) of about 4,000,000), 69.65 parts by weight of water, and zirconia beads having 4 times the weight of zinc oxide as a stirrer were placed in a polypropylene container with a lid (100 mL), covered with the lid, and then was dispersed for 2 hours with the paint conditioner. Thereafter, Aqueous Dispersion 3 was obtained by filtering through a 200-micron polyethylene mesh. The obtained Aqueous Dispersion 3 was allowed to stand at room temperature, and then the dispersion state over time was visually observed. As a result, Aqueous Dispersion 3 was in a uniform state even after 3 months. Further, when this Aqueous Dispersion 3 was applied to a latex sheet, a uniform film was obtained.

### (Example 4)

30 parts by weight of hydrous silica-treated zinc oxide fine particles (manufactured by Sakai chemical industry Co., Ltd., Finex52), 0.35 parts by weight of SACRAN (weight-average molecular weight (Mw) of about 12,000,000), 69.65 parts by weight of water, and zirconia beads having 4 times the weight of zinc oxide as a stirrer were placed in a polypropylene container with a lid (100 mL), covered with the lid, and then dispersed for 2 hours with the paint conditioner. Thereafter, Aqueous Dispersion 4 was obtained by filtering through a 200-micron polyethylene mesh. The obtained Aqueous Dispersion 4 was allowed to stand at room temperature, and then the dispersion state over time was visually observed. As a result, Aqueous Dispersion 4 was in a uniform state even after 3 months. Further, when this Aqueous Dispersion 4 was applied to a latex sheet, a uniform film was obtained.

### (Comparative Example 1)

30 parts by weight of untreated titanium oxide fine particles, 0.25 parts by weight of SACRAN (weight-average molecular weight (Mw) of about 4,000,000), 69.75 parts by weight of water, and zirconia beads having 4 times the weight of titanium oxide as a stirrer were placed in a polypropylene container with a lid (100 mL), covered with the lid, and then dispersed for 2 hours with the paint conditioner. Thereafter, Comparative Aqueous Dispersion 1 was obtained by filtering through a 200-micron polyethylene mesh. The obtained Comparative Aqueous Dispersion 1 was allowed to stand at room temperature, and then the dispersion state over time was visually observed. After one week, Comparative Aqueous Dispersion 1 was separated into two layers. Further, when this Comparative Aqueous Dispersion 1 was applied to a latex sheet, only a non-uniform film was obtained.

### (Comparative Example 2)

30 parts by weight of untreated zinc oxide fine particles, 0.35 parts by weight of SACRAN (weight-average molecular weight (Mw) of about 12,000,000), 69.65 parts by weight of water and zirconia beads having 4 times the weight of titanium oxide as a stirrer were placed in a polypropylene container with a lid (100 mL), covered with the lid, and then dispersed for 2 hours with the paint conditioner. Thereafter, Comparative Aqueous Dispersion 2 was obtained by filtering through a 200-micron polyethylene mesh. The obtained Comparative Aqueous Dispersion 2 was allowed to stand at room temperature, and then the dispersion state over time was visually observed. After one week, Comparative Aqueous Dispersion 2 was separated into two layers. Further, when this Comparative Aqueous Dispersion 2 was applied to a latex sheet, only a non-uniform film was obtained.

### (Comparative Example 3)

30 parts by weight of hydrous silica-treated titanium oxide fine particles, 0.25 parts by weight of sodium hyaluronate (weight-average molecular weight of about 1,600,000), 69.75 parts by weight of water, and zirconia beads having 4 times the weight of titanium oxide as a stirrer were placed in a polypropylene container with a lid (100 mL), covered with the lid, and dispersed for 2 hours with the paint conditioner. Thereafter, Comparative Aqueous Dispersion 3 was obtained by filtering through a 200-micron polyethylene mesh. The obtained Comparative Aqueous Dispersion 3 was allowed to stand at room temperature, and then the dispersion state over time was visually observed. As a result, a part of the dispersion of Comparative Aqueous Dispersion 3 was separated after one month. Further, when this Comparative Aqueous Dispersion 3 was applied to a latex sheet, only a non-uniform film was obtained.

### (Comparative Example 4)

30 parts by weight of hydrous silica-treated zinc oxide fine particles, 0.35 parts by weight of fucoidan (weight-average molecular weight of about 300,000), 69.65 parts by weight of water, and zirconia beads having 4 times the weight of zinc oxide as a stirrer were placed in a polypropylene container with a lid (100 mL), covered with the lid, and then dispersed for 2 hours with the paint conditioner. Thereafter, Comparative Aqueous Dispersion 4 was obtained by filtering through a 200-micron polyethylene mesh. The obtained Comparative Aqueous Dispersion 4 was allowed to stand at room temperature, and then the dispersion state over time was visually observed. As a result, a part of Comparative Aqueous Dispersion 4 was separated after one month. Further, when this Comparative Aqueous Dispersion 4 was applied to a latex sheet, only a film having partial unevenness was obtained.

### (Example 5)

2 parts by weight of cetostearyl alcohol, 1.2 parts by weight of glyceryl stearate, 0.6 parts by weight of polyoxyethylene 40 stearate, 0.2 parts by weight of steareth 20, 2 parts by weight of jojoba oil, and 3 parts by weight of lauryl laurate were mixed and dissolved by heating at 60 °C. to obtain Dissolved Matter 1. Next, Dissolved Matter 1 obtained above was added to a 60 °C solution obtained by adding 3 parts by weight of glycerin polyethylene glycol ether to 47 parts by weight of RO water. This solution was stirred with a homogenizer for 10 minutes, and then 40 parts by weight of Aqueous Dispersion 1 prepared in Example 1 was added, and stirring was performed with a homogenizer for 10 minutes. To the obtained dispersion, 1 part by weight of phenoxyethanol was added and kneaded to obtain a lotion. The lotion obtained as described above was evaluated for the SPF (Sun Protect Factor), the feeling upon application to the skin, the whiteness, and the squeaky feeling using the evaluation methods described below.

### <Evaluation Method of Cosmetic (Lotion) using Aqueous Dispersion>

### «Evaluation of SPF (SPF Evaluation)»

The SPF was evaluated using an SPF analyzer (UV-2000S, manufactured by Labsphere Inc., USA).

### [Evaluation Criteria]

A: SPF of 30 or more.
B: SPF of 25 or more and less than 30.
C: SPF of 20 or more and less than 25.
D: SPF of less than 20.

### <<Feeling upon Application to Skin (Skin Feel Characteristics)>>

For the evaluation of the feeling upon application to the skin, the prepared lotion was placed on the tip of the index finger and evenly applied to the back side of the arm.

### [Evaluation Criteria]

A: Four or more persons out of five persons answered that the feeling was good.
B: Three persons out of five persons answered that the feeling was good.
C: Two persons out of five persons answered that the feeling was good.
D: One person out of five persons answered that the feeling was good, or there was no answer that the feeling was good.

### <<Whiteness>>

The whiteness was evaluated according to the following criteria in comparison with the skin after application to the skin.

### [Evaluation Criteria]

A: Whiteness was not conspicuous.
B: Whiteness was slightly conspicuous.
C: Whiteness was conspicuous.

### <<Squeaky Feeling>>

For the squeaky feeling, the prepared lotion was placed on the tip of the index finger and applied evenly to the back side of the arm.

### [Evaluation Criteria]

A: Four or more persons out of five persons answered that the squeaky feeling was not felt.
B: Three persons out of five persons answered that the squeaky feeling was not felt.
C: Two persons out of five persons answered that the squeaky feeling was not felt.
D: One person out of five persons answered that the squeaky feeling was not felt, or there was no answer that the squeaky feeling was not felt

The evaluation results of the SPF, the feeling upon application to the skin, the whiteness, and the squeaky feeling for the lotion of Example 5 prepared using the aqueous dispersion of Example 1 are shown in Table 1 below.

### (Example 6)

A lotion was obtained in the same manner as in Example 5, except that Aqueous Dispersion 1 was changed to Aqueous Dispersion 2 obtained in Example 2.

The lotion obtained in Example 6 was evaluated for the SPF, the feeling upon application to the skin, the whiteness, and the squeaky feeling in the same manner as in Example 5. The evaluation results are shown in Table 1 below.

### (Example 7)

A lotion was obtained in the same manner as in Example 5, except that Aqueous Dispersion 1 was changed to Aqueous Dispersion 4 obtained in Example 4.

The lotion obtained in Example 7 was evaluated for the SPF, the feeling upon application to the skin, the whiteness, and the squeaky feeling in the same manner as in Example 5. The evaluation results are shown in Table 1 below.

### (Comparative Example 5)

30 parts by weight of hydrous silica-treated titanium oxide fine particles, 70 parts by weight of water, and zirconia beads having 4 times the weight of titanium oxide as a stirrer were placed in a polypropylene container with a lid (100 mL), covered with the lid, and then dispersed for 2 hours with the paint conditioner. Thereafter, Comparative Aqueous Dispersion 5 was obtained by filtering through a 200-micron polyethylene mesh.

A lotion was obtained in the same manner as in Example 5, except that Aqueous Dispersion 1 was changed to Comparative Aqueous Dispersion 5 obtained in Comparative Example 5.

The lotion obtained in Comparative Example 5 was evaluated for the SPF, the feeling upon application to the skin, the whiteness, and the squeaky feeling in the same manner as in Example 5. The evaluation results are shown in Table 1 below.

### (Comparative Example 6)

2 parts by weight of cetostearyl alcohol, 1.2 parts by weight of glyceryl stearate, 0.6 parts by weight of polyoxyethylene 40 stearate, 0.2 parts by weight of steareth 20, 2 parts by weight of jojoba oil, and 3 parts by weight of lauryl laurate were mixed and dissolved by heating at 60 °C to obtain Dissolved Matter 2.

Next, Dissolved Matter 2 obtained above was added to a 60 °C solution obtained by adding 3 parts by weight of glycerin polyethylene glycol ether to 27.5 parts by weight of RO water. This solution was stirred with a homogenizer for 10 minutes, and then 40 parts by weight of Comparative Aqueous Dispersion 5 prepared in Comparative Example 5 was added, and stirring was performed with a homogenizer for 10 minutes. Further, 20 parts by weight of 0.5 % SACRAN aqueous solution was added, and stirring was performed with a homogenizer for 10 minutes.

To the obtained dispersion, 1 part by weight of phenoxyethanol was added and kneaded to obtain a lotion.

The lotion obtained in Comparative Example 6 was evaluated for the SPF, the feeling upon application to the skin, the whiteness, and the squeaky feeling in the same manner as in Example 5. The evaluation results are shown in Table 1 below.

### (Comparative Example 7)

A lotion was obtained in the same manner as in Comparative Example 5, except that the hydrous silica-treated zinc oxide fine particles were used instead of the hydrous silica-treated titanium oxide fine particles.

The lotion obtained in Comparative Example 7 was evaluated for the SPF, the feeling upon application to the skin, the whiteness, and the squeaky feeling in the same manner as in Example 5. The evaluation results are shown in Table 1 below.

**[Table 1]**

| | Example 5 | Example 6 | Example 7 | Comparative Example 5 | Comparative Example 6 | Comparative Example 7 |
|---|---|---|---|---|---|---|
| SPF Evaluation | A | A | B | C | C | C |
| Skin Feel Characteristics | A | A | B | C | B | C |
| Whiteness | A | A | A | C | B | C |
| Squeaky Feeling | A | A | B | D | C | D |

From the results of Examples 1 to 4 and Comparative Examples 1 to 4, it was found that the aqueous dispersion including the polysaccharide having a sulfate group and the hydrophilization treatment-inorganic powder was excellent in dispersion stability and also excellent in coating film formability.

In addition, from the results of Examples 5 to 7 and Comparative Examples 5 to 7, it was found that the aqueous dispersion including the polysaccharide having a sulfate group and the hydrophilization treatment-inorganic powder effectively contributes to the improvement of the SPF value, the skin feel characteristics, and the like of the cosmetic.

## Claims

1. An aqueous dispersion comprising:
a polysaccharide (A) having a sulfate group; and
a hydrophilization treatment-inorganic powder (B).

2. The aqueous dispersion according to claim 1, wherein the polysaccharide (A) having a sulfate group is a polysaccharide having a weight-average molecular weight of 3,500,000 to 20,000,000.

3. The aqueous dispersion according to claim 1 or 2, wherein the hydrophilization treatment is a hydrophilization treatment of coating a part or all of a surface of the inorganic powder with one or more surface treatment agents selected from silica, hydrous silica, hydrous alumina, zirconia, and the like.

4. The aqueous dispersion according to any one of claims 1 to 3, wherein the inorganic powder is one or more selected from titanium oxide or zinc oxide.

5. The aqueous dispersion according to any one of claims 1 to 4, wherein the hydrophilization treatment-inorganic powder (B) is contained in a range of 10 to 200 parts by weight with respect to 1 part by weight of the polysaccharide (A) having a sulfate group in the aqueous dispersion.

6. The aqueous dispersion according to any one of claims 1 to 5, wherein the polysaccharide (A) having a sulfate group is a polysaccharide obtained from Suizenji Nori.

7. A cosmetic comprising the aqueous dispersion according to any one of claims 1 to 6.

8. The cosmetic according to claim 7, wherein the cosmetic is a sunscreen cosmetic or a moisturizing cosmetic.
